(19) Europäisches Patentamt
European Patent Office
Office européen des brevets

(11) Publication number : **0 346 713 B1**

(12) # EUROPEAN PATENT SPECIFICATION

(45) Date of publication of patent specification : **16.09.92 Bulletin 92/38**

(51) Int. Cl.$^5$ : **C07C 257/18,** C08K 5/29

(21) Application number : **89110111.5**

(22) Date of filing : **05.06.89**

(54) **Benzamidines useful as ultraviolet light absorbers.**

(30) Priority : **15.06.88 US 207088**
**11.04.89 US 336286**

(43) Date of publication of application : **20.12.89 Bulletin 89/51**

(45) Publication of the grant of the patent : **16.09.92 Bulletin 92/38**

(84) Designated Contracting States : **AT BE CH DE FR GB IT LI NL**

(56) References cited :
**GB-A- 1 267 009**
**US-A- 4 085 062**
**TETRAHEDRON, vol. 27, no. 16, August 1971, pages 3811-3819, Pergamon Press, GB; Y. MORI et al.: "Organic syntheses by meansof metal complexes-VI"**

(56) References cited :
**CHEMICAL ABSTRACTS, vol. 87, part 5, 1st August 1977, page 428, abstract no. 38331t, Columbus, Ohio, US; J. SEVCIK: "Amidines. IV. Spectrophotometric study of N,N'-di- and N,N'-trisubstituted benzamidine in the UV region",**

(73) Proprietor : **L. GIVAUDAN & CIE Société Anonyme**
**CH-1214 Vernier-Genève (CH)**

(72) Inventor : **Cohen, Isaac D.**
**1885 East 13th. Street**
**Brooklyn, N.Y. 11229 (US)**
Inventor : **Virgilio, Joseph A.**
**14 Evelyn Terrace**
**Wayne, N.J. 07470 (US)**

(74) Representative : **Urech, Peter, Dr. et al**
**Grenzacherstrasse 124 Postfach 3255**
**CH-4002 Basel (CH)**

**EP 0 346 713 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention discloses novel ultraviolet absorbing N,N'-diarylbenzamidines useful for protecting materials against the degradative effects of ultraviolet light.

Various polymers, plastics, resins, cosmetics, dyes, pigments, lacquers, varnishes, textiles, etc., which are subject to photodegradation by UV-radiation, can be protected by incorporating therein suitable UV-light absorbing agents which will absorb the harmful rays and convert them to relatively harmless forms of energy. To be effective, the light absorbing agent must absorb light efficiently in the ultraviolet portion of the sun's rays which reach the earth, i.e., the range of 280 to 400 nanometers. The agent should also be stable to UV-radiation, compatible with and stable in the medium in which it is incorporated, possess little or no color, be non-toxic, thermally stable, and have low volatility.

Arylsubstituted benzamidines, namely N-mono-arylsubstituted benzamidines and their UV-absorption data are known (see Monatshefte für Chemie, 100, 1307-9 (1969). These known compounds differ in structure by at least a phenyl ring. From the UV-data disclosed in the reference it is clear that one would not expect the substituted benzamidines disclosed therein to have any commercial utility as sunscreen agents. To be effective, a light absorbing agent should preferably have continuous high absorptivity over the range 280 to 400 nanometers, i.e., the ultraviolet portion of the sun's rays which reach earth. Figure 1 of the reference, shows an absorption curve which is said to be typical for the disclosed mono-substituted benzamidines. This Figure teaches that the UV absorption of the compounds drops off rapidly after maximum absorption (λ max) and, for N-phenylbenzamidine, approaches zero before 300 nanometers. This sharp drop in absorption and lack of absorption over the critical range of 280-400 nanometers would appear to make the disclosed mono-substituted compounds ineffective as commercial sunscreen agents.

In accordance with the present invention, there are provided N,N'-diarylbenzamidines of the formula

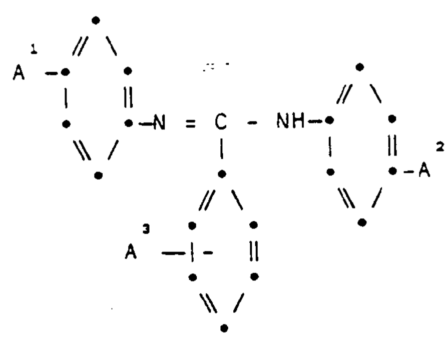

I

wherein

$A^1$ and $A^2$ may be the same or different and are selected from the group consisting of acid, ester, amide, cyano and aryl radicals, and $A^3$ is selected from the group consisting of hydrogen, hydroxy, cyano, nitro, halogen, ether, acid, ester, amide, phenyl and alkyl radicals.

The benzamidines of this invention are particularly valuable inasmuch as they absorb radiation in both the UV-A and UV-B regions and are particularly effective in the range of 280 to 375 nanometers. They are stable to photodegradation and thermal degradation. They are colorless or nearly colorless at the concentrations used, which makes them particularly valuable in retarding photodegradation in those cases where color could be a problem, such as in clear plastics. They are resistant to attack by polar solvents, such as methanol, ethanol, isopropanol, and the like, which makes them particularly valuable in applications based on alcoholic and hydro-alcoholic solutions, suspensions, emulsions and the like.

As indicated above, the novel benzamidines of this invention have the formula

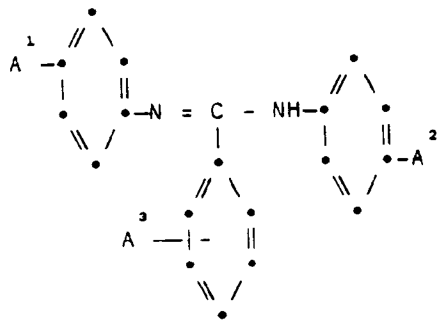

I

wherein
$A^1$ and $A^2$ may be the same or different and are selected from the group consisting of acid, ester, amide, nitrile and aryl radicals, and $A^3$ is selected from the group consisting of hydrogen, cyano, nitro, halogen, ether, acid, ester, amide, phenyl and alkyl radicals.

While $A^1$ and $A^2$ can be any suitable electron withdrawing group, those compounds wherein $A^1$ and $A^2$ are acid, ester, amide, cyano or aryl radicals are the more readily available. Preferred are compounds wherein $A^1$ and $A^2$ are selected from the group consisting of $-COOR^1$, $-CONR^1R^2$, -CN and $-C_6H_5$. The nature of $R^1$ and $R^2$ has very little influence on the UV-absorbing properties of the benzamidine I and may be hydrogen or any suitable alkyl, aryl or aralkyl group. The more readily available esters and amides wherein $R^1$ or $R^2$ is a lower alkyl group of from one to ten carbon atoms are preferred with methyl and ethyl being especially preferred.

The most preferred compounds are those wherein $A^1$ and $A^2$ are methyl or ethyl ester groups.

While $A^1$ and $A^2$ may be the same or different, it is preferred to have $A^1$ and $A^2$ be the same for simplicity and economy of synthesis.

The nature of $A^3$ does not appear to have a large influence on the UV-absorbing properties and may be any suitable group, e.g. hydrogen, a cyano, a halogen, a nitro group, an ether, an acid, an ester, an amide, phenyl radical, an alkyl radical or the like. The more readily available starting materials are those wherein $A_3$ is selected from the group consisting of -H, -OH, $-NO_2$, -Cl, -CN, $-C_6H_5$, alkyl groups of one to five carbon atoms and ether radicals of the type -OR wherein R is an alkyl group of from one to ten carbon atoms. Especially preferred are those benzamidines wherein $A^3$ is chosen from the group consisting of hydrogen and methoxy.

A number of methods known in the art can be adapted to prepare the novel N,N'-diarylbenzamidines of the present invention [See for example A.C. Hontz, E.C. Wagner, Org. Syn., Coll. Vol. IV, John Wiley & Sons, Inc., New York, 383 (1963); S.P. Joshi, A.P. Khanolkar, T.S. Wheeler, J. Chem. Soc., 793 (1936)].

The convenient method comprises reacting an anilide carrying the p-substituent $A^1$ with a benzotrihalide carrying the substituent $A^3$, or reacting a benzoyl halide carrying the p-substituent $A^3$ with an anilide carrying the p-substituent $A^2$ and reacting the amide so obtained with a further anilide carrying the p-substituent $A^1$.

Scheme I below sets forth a convenient method for preparing those compounds of formula I wherein $A^2$ is identical to $A^1$.

SCHEME I

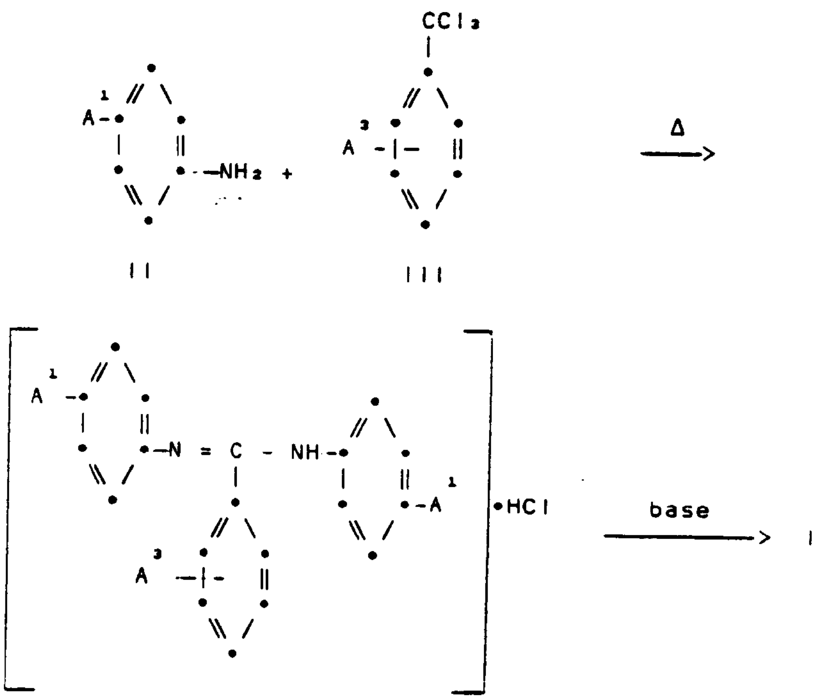


II III

In this process, it is preferred to react about two moles of the appropriate substituted aniline II with each mole of the appropriate substituted benzotrichloride III used. In the preferred process of this invention the reactants II and III above, are heated to reflux in an aromatic solvent such as toluene or xylene for about twelve to twenty-four hours. The reaction mixture is then cooled to about 0°C. The hydrochloride salt I′ precipitates and can be removed by simple filtration. In those instances wherein the hydrochloride salt itself is preferred as the UV-absorbing agent, it can be used as such. In most instances, however, it is preferred to use the benzamidine per se and the benzamidine may be liberated by treating the hydrochloride salt with dilute base.

The use of a solvent is not essential. The reaction may be carried out in the absence of solvent. The addition of an aromatic solvent to the reaction mixture does, however, facilitate control of the exothermic condensation and is generally preferred. The use of refluxing xylene for this reaction is especially preferred.

Those compounds of formula I wherein $A^1$ is not the same as $A^2$ may be conveniently prepared, as illustrated in Scheme II.

## SCHEME II

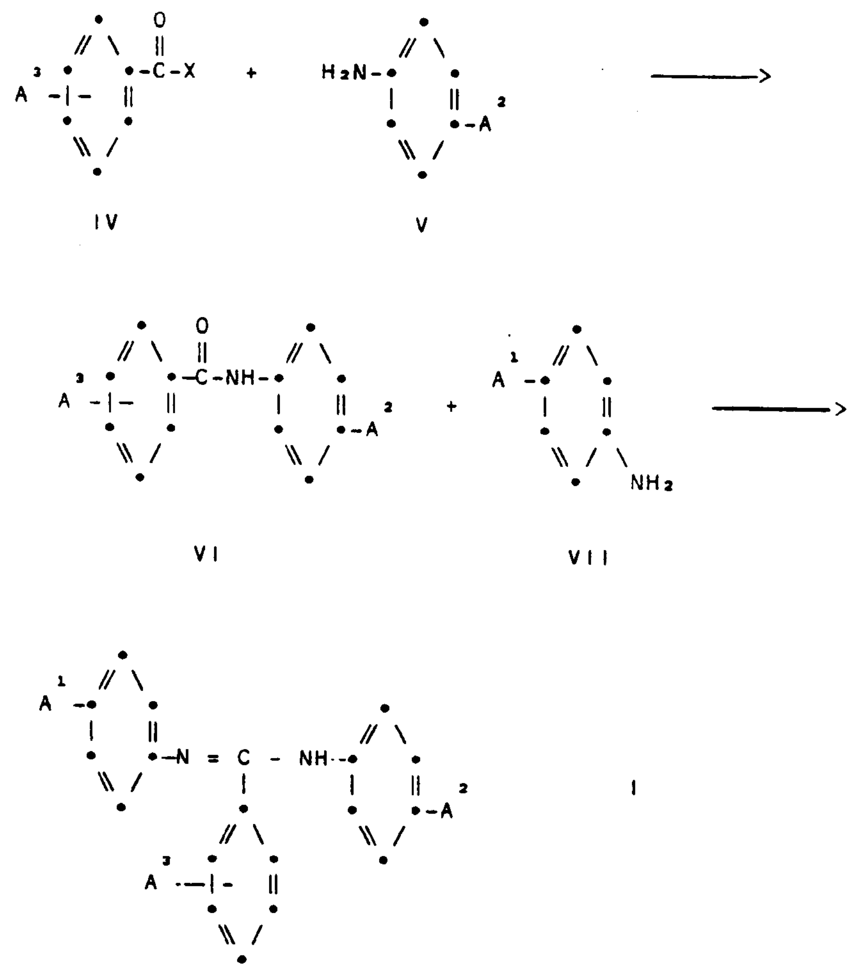

IV

V

VI

VII

I

As shown in Scheme II, the appropriate aniline, V, is reacted with the appropriate benzoyl halide, IV, in approximately equimolar ratios to form the benzamide derivative, VI. This benzamide derivative, VI, is subsequently condensed with a second and different aniline, VII, in the presence of an activating agent, such as $PCl_5$, $POCl_3$, or the like,to provide the hydrochloride salt of I. The hydrochloride salt is usually converted to I during the basic workup procedure. Typical procedures are provided in the examples.

Of course, the method of Scheme II could also be used to prepare those benzamidines wherein $A^1$ and $A^2$ are identical, simply by using the same aniline for both steps, i.e., V and VII would be the same. In this case, steps 1 and 2 may be run concurrently by allowing approximately two moles of the aniline, V, to react with one mole of the benzoyl halide, IV, in the presence of the activating agent. Alternatively, the acid precursor of IV may be used, in which case the benzoyl halide is formed in situ. The methods of preparation, which are not critical to this invention, are further illustrated in the examples provided herein.

Table I lists the strong ultraviolet-absorbing properties of several benzamidines prepared by either Scheme I or Scheme II. The absorptions were determined by dissolving the compounds in isopropanol and recording the spectrum on an ultraviolet spectrophotometer. Table I lists the wavelength of maximum absorption ($\lambda$ max), the intensity of this absorption as molar extinction coefficient ($\varepsilon$ max), and the $\lambda$ range where $\varepsilon$ = 5,000 or greater. Also, given in Table I, are the UV characteristics of two hydrochloride salts. [The benzamidines of this invention have a basic nitrogen and can be converted into salts of, e.g. mineral acids, e.g. into their hydrohalide salts, if desirable, for certain applications. It is also possible to isolate the hydrochloride salt from the reaction medium since in both Scheme I and Scheme II, as discussed above, the hydrochloride salt is formed as a reaction intermediate which must be treated with base to provide the benzamidine per se.]

TABLE I

| Entry | Scheme | Compound | λ max (nm) | ε max | λ Range* (nm) ε>5,000 |
|---|---|---|---|---|---|
| 1 | I | N,N'-bis(4-methoxy-carbonylphenyl)benzamidine | 318 | 27,800 | 280-374 |
| 2 | I | N,N'-bis(4-ethoxy-carbonylphenyl)benzamidine | 320 | 26,300 | 280-373 |
| 3 | I | N,N'-bis(4-[2-ethylhexyl]-oxycarbonylphenyl)benzamidine | 320 | 23,000 | 280-375 |
| 4 | I | N,N'-bis(4-n-octylcarbamyl-phenyl)benzamidine | 296 | 21,800 | 280-368 |
| 5 | II | N-(4-biphenyl)-N'-(4-ethoxy-carbonylphenyl)benzamidine | 288 | 26,400 | 280-378 |
| 6 | II | N,N'-bis(4-ethoxycarbonyl-phenyl)-2-methoxybenzamidine | 318 | 31,300 | 280-366 |
| 7 | II | N,N'-bis(4-ethoxycarbonyl-phenyl)-4-methoxybenzamidine | 322 | 26,200 | 280-370 |
| 8 | II | N,N'-bis(4-ethoxycarbonyl-phenyl)-4-cyanobenzamidine | 302 | 27,600 | 280-371 |
| 9 | II | N,N'-bis(4-ethoxycarbonyl-phenyl)-4-phenylbenzamidine | 292 | 28,800 | 280-385 |
| 10 | I | N,N'-bis(4-ethoxycarbonyl-phenyl)-2-chlorobenzamidine | 302 | 25,000 | 280-350 |
| 11 | II | N,N'-bis(4-ethoxycarbonyl-phenyl)-3-chlorobenzamidine | 300 | 24,200 | 280-368 |
| 12 | II | N,N'-bis(4-ethoxycarbonyl-phenyl)-4-chlorobenzamidine | 318 | 24,000 | 280-372 |
| 13 | II | N,N'-bis(4-ethoxycarbonyl-phenyl)-4-methylbenzamidine | 315 | 19,100 | 280-369 |
| 14 | II | N,N'-bis(4-ethoxycarbonyl-phenyl)-4-t-butylbenzamidine | 322 | 24,500 | 280-376 |
| 15 | II | N,N'-bis(4-ethoxycarbonyl-phenyl)-4-nitrobenzamidine | 304 | 36,200 | 280-378 |
| 16 | I | N,N'-bis(4-ethoxycarbonyl-phenyl)-benzamidine hydrochloride | 320 | 27,000 | 280-376 |
| 17 | II | N,N'-bis(4-ethoxycarbonyl-phenyl)-4-nitrobenzamidine hydrochloride | 302 | 32,300 | 280-367 |

*Only wavelengths above 280nm are included.

All of the benzamidines set forth in Table I can be characterized by their high absorptivity and the broad range over which the absorptivity is effective. High absorption over a broad range is an especially desirable property of a commercial UV-screening agent since, in practice, it is often difficult to determine the wavelength which will be most deleterious to the material to be protected. The benzamidines of Table I also show excellent resistance to photochemical and thermal degradation.

Sensitive materials can be protected from the harmful effects of UV-light by incorporating an N,N′-diarylbenzamidine of the invention into the UV-sensitive material or into materials used to coat or protect UV-sensitive materials. They can be admixed with dyes or cosmetics to preserve the integrity of these materials. Incorporation into plastics prevents discoloration, etc. which may occur in the absence of an effective UV-absorber.

UV-sensitive materials can also be protected from the harmful rays by coating with a material containing the UV-screening agent. The compositions of this invention can also be incorporated into lotions to protect the skin from UV-radiation, or incorporated into plastic containers or container coatings which will serve to protect the contents of such containers from the harmful effects of UV-radiation.

The effective amounts of screening agent necessary for each application would be dependent on that application and determinable by those skilled in the art. For most applications, a preferred range of 0.01 wt percent to 4 wt percent is effective, with 0.05 wt percent to 2 wt percent being especially preferred. However, in special formulations where higher concentrations are needed, amounts as high as 8 wt percent may be used. Such high amounts are often required in cosmetic formulations.

The following examples provide a more detailed explanation of the invention and are intended as illustrations and not limitations of the invention. For examples 1 through 5, melting points were determined in open-ended capillary tubes and are not corrected; 60 MHz, $^1$H-NMR chemical shifts are reported downfield from internal tetramethylsilane (TMS) on the w scale; and IR absorptions are reported in reciprocal centimeters.

Example 1 - Scheme I: Preparation of N,N′-Bis(4-ethoxycarbonyl-phenyl)benzamidine, 2 (see table I).

a. N,N′-Bis(4-ethoxycarbonylphenyl)benzamidine hydrochloride, 16:

To a stirred suspension of ethyl 4-aminobenzoate (165.0 g; 1.0 mole) in xylene (465 mL) was added α,α,α-trichlorotoluene (100.7 g; 0.52 mole). The mixture was allowed to reflux for 18h, then was cooled to 0°C and filtered. The resultant precipitate was washed with ice-cold xylene (2x70 mL), triturated with hot toluene and dried to yield the salt (89.8g; 39.7%) as white crystals:
mp 214.0-215.5°C. $^1$H-NMR ($CDCl_3$) 8.0-6.9 (m,14H), 4.3 (q,4H, J = 7Hz), 1.4 (t,6H, J = 7Hz); IR ($CHCl_3$) 1715, 1600;MS (m/e) 416 ($M^+$-HCl), 252 (base).

b. N,N′-Bis(4-ethoxycarbonylpheny)benzamidine, 2:

The salt prepared as in part a (53.6g), was added to a vigorously stirred mixture of ice-water (500 mL),concentrated ammonium hydroxide (20 mL), and toluene (100 mL).After filtering and drying one obtains the benzamidine (37.1 g; 75.3%) as a pale-yellow solid: mp 170-172°C. $^1$H-NMR ($CDCl_3$) 8.0-7.0 (m,14H), 4.2 (q,4H, J = 6Hz), 1.3 (t,6H, J = 6Hz); IR ($CHCl_3$) 3330, 1700, 1590; MS (m/e) 416 ($m^+$), 105 (base).

Example 2 - Scheme II: Preparation of N,N′-Bis(4-ethoxycarbonylphenyl)-4-nitrobenzamidine, 15.

a. N,N′-Bis(4-ethoxycarbonylphenyl)-4-nitrobenzamidine hydrochloride, 17.

To a stirred suspension of ethyl 4-N-(4-nitrobenzoyl)-amino benzoate (29.8g; 0.10 mole) in dichloromethane (300 ml) was added phosphorus pentachloride (22.2g; 0.11 mole) in small portions. The reaction mixture was allowed to reflux for 3h; ethyl 4-aminobenzoate (16.5g; 0.10 mole) was then added in small portions.The mixture was allowed to stir at reflux for 3h and at room temperature overnight, then quenched with ice-water (300 ml) and neutralized with concentrated ammonium hydroxide. The organic layerwas concentrated to an orange mass, which was triturated with hot toluene, filtered, and dried to yield the salt (34.4g; 78.5%) as an off-white solid: mp 238-242°C. $^1$H-NMR (DMSO-d6) 8.4-7.0(m, 14H), 4.2 (q, 4H, J=7Hz), 1.3 (t, 6h, J=7Hz); IR (Nujol) 1725, 1550; MS (m/e) 461 ($M^+$-HCl), 36 (base).

b. N,N′-Bis (4-ethoxycarbonylphenyl)-4-nitrobenzamidine, 15.

The salt prepared as in part a (1.0 g) was dissolved in warm methanol (20ml) with vigorous stirring. The solution was brought to pH 8 by dropwise addition of pyridine, then was cooled and filtered. The resultant precipitate was washed with methanol and dried to yield the benzamidine (0.8g; 87.0%) as yellow crystals: mp 202-205°C. $^1$H-NMR ($CDCl_3$) 8.1-7.0 (m, 13H), 4.3(q, 4H, J=7Hz), 1.3 (t, 6H, J=7Hz); IR (Nujol) 3300, 1710, 1680, 1590; MS (m/e) 461 ($M^+$), 297 (base).

Example 3 - Scheme II: Preparation of N,N′-Bis(4-ethoxycarbonyl-phenyl)-2-methoxybenzamidine, 6.

To a stirred solution of ethyl 4-N-(2-methoxybenzoyl)amino-benzoate (15.0g; 0.05 mole) in dichloromethane (150 mL) was added phosphorus pentachloride (11.1 g; 0.053 mole) in small portions. After the addition was completed, the mixture was allowed to reflux for 1h. Ethyl 4-aminobenzoate (8.3 g; 0.050 mole) was added in small portions and the solution was allowed to stir at reflux for 3h, and at room temperature for 18h. The reaction mixture was poured into ice-water (150 mL) and neutralized with concentrated ammonium hydroxide. The organic phase was concentrated to a yellow solid, which was triturated with wet diethyl ether with gentle warming to yield a white solid (28g). Recrystallization from isopropanol gave the benzamidine (15.5g; 68.9%.) as white crystals: mp 163-164°C. $^1$H-NMR ($CDCl_3$) 7.9-6.6 (m,13H), 4.2 (q,4H, J = 7Hz), 3.6 (s,3H), 1.3 (t,6H, J = 7Hz); IR (Nujol) 3370, 1700, 1585;MS (m/e) 446 ($M^+$) 282 (base).

Example 4 - Scheme II: Preparation of N-(4-Biphenyl)N′-(4-ethoxy-carbonylphenyl)benzamidine, 5.

To a stirred solution of ethyl 4-(N-benzoyl)aminobenzoate (13.5g; 0.05 mole) in chloroform (150 mL) was added phosphorus pentachloride (11.5g; 0.055 mole) in small portions. The reaction mixture was allowed to reflux for 1h, after which 4-aminobiphenyl(8.5 g; 0.05 mole) was added in small portions. The mixture was refluxed for 2h, then poured into ice-water (150mL) and neutralized with concentrated ammonium hydroxide. The organic phase was filtered and concentrated to a yellow solid. Recrystallization from toluene yielded the benzamidine 3.5g; 16.7%) as pale-yellow crystals: mp 155-158°C. $^1$H-NMR ($CDCl_3$) 8.0-6.8 (m,19H), 4.2 (q,2H, J = 7Hz), 1.3 (t,3H, J = 7Hz); IR ($CHCl_3$) 3430, 1695, 1585; MS (m/e) 420 ($M^+$), 252 (base).

Example 5 Scheme II: Preparation of N,N′-Bis (4-ethoxycarbonylphenyl)-4-methoxybenzamidine, 7.

Phosphorus oxychloride (76.8g, 0.5 mol) was added dropwise to a stirred solution of 4-methoxybenzoic acid (40g, 0.26 mol), benzocaine (82.4g, 0.5 mol) and toluene (500 ml) at 80°C. The reaction was refluxed for 24 hrs. then quenched with 30% sodium hydroxide. The organic phase was then separated, cooled and the resulting precipitate recrystallized from toluene to yeld the benzamidine (48g, 43%) as a pale yellow solid: mp 145°-146°C. H-NMR ($CDCl_3$) 1.36 (+,6H), 3.78 (s,3H), 4.29-4.37 (Q,4H), 6.7-8.0 (m,13H); IR ($CHCl_3$) 1710, 1690, 1640, 1605, 1590, 1260; MS (m/e) 426 ($m^+$) 282 (base).

Example 6 - Preparation of additional N,N′-diarylbenzamidines reported in Table I.

a. N,N′-Bis(4-methoxycarbonylphenyl)benzamidine (1) was prepared from methyl 4-aminobenzoate and α,α,α-tri-chlorotoluene; pale-yellow crystals: mp 180-182°C. $^1$H-NMR ($CDCl_3$) 8.0-7.0 (m,14H), 3.8 (s,6H); IR ($CDCl_3$) 3330, 1705, 1585; MS (m/e) 388 ($M^+$), 238 (base).
b. N,N′-Bis[4-(2-ethylhexyl)oxycarbonylphenyl]benzamidine (3) was prepared from 2-ethylhexyl 4-aminobenzoate and α,α,α-tri-chlorotoluene; viscous, gold oil: $^1$H-NMR ($CDCl_3$) 7.9-6.8 (m,14H), 4.1 (d,4H, J = 4Hz), 1.9-0.8 (m,30H); IR (Neat) 3330, 1710, 1585; MS (m/e) 584 ($M^+$), 336 (base).
c. N,N′-Bis(4-n-octylcarbamylphenyl)benzamidine (4) was prepared from 4-amino-N-n-octylbenzamide and α,α,α-trichloro-toluene; pale-yellow oil: $^1$H-NMR ($CDCl_3$) 7.7-6.9 (m,14H), 6.2 (br t,2H, J = 6Hz), 3.3 (br q,4H, J = 6Hz), 1.7-0.7 (m,30H); IR ($CHCl_3$) 3430, 1640, 1590; MS (m/e) 582 ($M^+$), 335 (base).
d. N,N′-Bis(4-ethoxycarbonylphenyl)-4-cyanobenzamidine (8) was prepared from ethyl 4-[N-(4-cyanobenzoyl)]-aminobenzoate, phosphorus pentachloride, and ethyl 4-aminobenzoate; pale-yellow crystals: mp 205-213°C. $^1$H-NMR (DMSO-d6), 8.1-7.4 (m,13H), 4.2 (q,4H, J = 7Hz, 1.3 (t,6H, J = 7Hz); IR ($CHCl_3$) 3320, 1705, 1590; MS (m/e) 441 ($M^+$), 277 (base).
e. N,N′-Bis(4-ethoxycarbonylphenyl)-4-phenylbenzamidine (9) was prepared from ethyl 4-[N-(4-phenylbenzoyl)]-aminobenzoate, phosphorus oxychloride, and ethyl 4-aminobenzoate; pale-yellow solid: mp 152-160°C. $^1$H-NMR ($CDCl_3$) 8.0-6.9 (m,18H), 4.2 (q,4H, J = 7Hz), 1.3 (t,6H, J = 7Hz); IR ($CHCl_3$) 3420, 1700, 1590; MS (m/e) 492 ($M^+$), 328 (base).
f. N,N′-Bis(4-ethoxycarbonylphenyl)-2-chlorobenzamidine (10) was prepared from ethyl 4-aminobenzoate and α,α,α,2-tetrachlorotoluene; tan solid: mp 98-108°C. $^1$H-NMR ($CDCl_3$) 8.3-6.4 (m,13H), 4.2 (q,4H, J = 7Hz); 1.3 (t,6H, J = 7Hz); IR ($CHCl_3$) 3360, 1695, 1600, 1580; MS (m/e) 450, 452 ($M^+$), 286 (base).
g. N,N′-Bis(4-ethoxycarbonylphenyl)-3-chlorobenzamidine (11) was prepared from ethyl 4-[N-(3-chlorobenzoyl)]-aminobenzoate, phosphorus pentachloride, and ethyl 4-aminobenzoate; yellow crystals: mp 160-170°C. $^1$H-NMR ($CDCl_3$) 8.0-6.8 (m,13H), 4.2 (q,4H, J = 7Hz), 1.3 (t,6H, J=7Hz);IR ($CDCl_3$) 3330, 1705, 1590; MS (m/e) 450, 452 ($M^+$),286 (base).

h. N,N′-Bis(4-ethoxycarbonylphenyl)-4-chlorobenzamidine (12) was prepared from ethyl 4-[N-(4-chlorobenzoyl)] aminobenzoate, phosphorus pentachloride, and ethyl 4-aminobenzoate; white crystals: mp 171.0-172.5°C. $^1$H-NMR ($CDCl_3$) 8.0-6.9 (m,13H), 4.2 (q,4H, J = 7Hz), 1.5 (t,6H, J = 7Hz); IR ($CHCl_3$) 3420, 3330, 1705, 1590; MS (m/e) 450, 452 ($M^+$), 286 (base).

i. N,N′-Bis(4-ethoxycarbonylphenyl)-4-methylbenzamidine (13) was prepared from ethyl 4-[N-(4-toluyl)]-aminobenzoate, phosphorus oxychloride, and ethyl 4-aminobenzoate; pale-yellow solid: mp 246-260°C. $^1$H-NMR ($CDCl_3$) 8.1-6.3 (m,13H), 4.3 (q,4H, J = 7Hz), 2.3 (s,3H), 1.3 (t,6H, J = 7Hz); IR ($CHCl_3$) 3420, 1710, 1590; MS (m/e) 430 ($M^+$), 266 (base).

j. N,N′-Bis(4-ethoxycarbonylphenyl)-4-t-butylbenzamidine (14) was prepared from ethyl 4-[N-(4-t-butylbenzoyl)]-aminobenzoate, phosphorus pentachloride, and ethyl 4-aminobenzoate; yellow solid: mp 212-219°C. $^1$H-NMR ($CDCl_3$) 8.0-7.0 (m,12H), 6.0 (br s,$^1$H), 4.3 (q,4H,J = 7Hz), 1.4 (t,6H, J = 7Hz), 1.3 (s,9H); IR (Nujol) 3330, 1710, 1590; MS (m/e) 472 ($M^+$), 308 (base).

## Claims

1. A compound of the formula

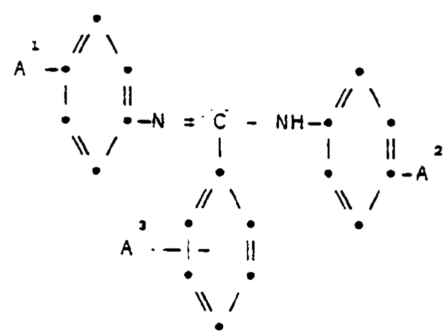

I

wherein
$A^1$ and $A^2$ may be the same or different and represent an acid, ester, amide, cyano or aryl radical, $A^3$ represents hydrogen, alkyl, phenyl, hydroxy, halogen, nitro, cyano or represents an ether, ester, acid or amide radical, or a salt thereof.

2. A compound according claim 1, wherein $A^1$ and $A^2$ may be the same or different and represent $-COOR^1$, $-CONR^1R^2$, -CN or $-C_6H_5$, $A^3$ represents -H, -OH, -OR, -Cl, $-NO_2$, -CN,$-C_6H_5$, or an alkyl group of one to five carbon atoms, $R^1$ and $R^2$ may be the same or different and represent hydrogen or an alkyl group of one to ten carbon atoms, and R represents an alkyl group of one to ten carbon atoms.

3. A compound according to claim 2, wherein $R^1$ and $R^2$ are methyl or ethyl.

4. A compound according to claim 2 wherein $A^1$ and $A^2$ are both $-COOR^1$.

5. A compound according to claim 4 wherein $A^3$ is hydrogen or -OR.

6. A compound according to claim 5 wherein $R^1$ is methyl or ethyl.

7. A compound according to claim 6 wherein $A^3$ is hydrogen or methoxy.

8. The compound according to claim 7 wherein $R^1$ is ethyl and $A^3$ is hydrogen.

9. A compound according to claim 7 wherein $R^1$ is ethyl and $A^3$ is methoxy.

10. N,N′-bis-(4-Ethoxycarbonylphenyl)-2-methoxybenzamidine.

11. N,N′-bis-(4-Ethoxycarbonylphenyl)-4-methoxybenzamidine.

12. A compound of the formula

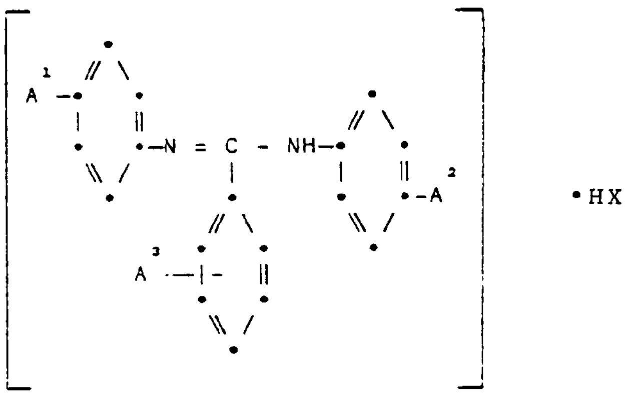

I'

wherein
$A^1$ and $A^2$ represent an acid, ester, amide, cyano or aryl radical, $A^3$ represents hydrogen, alkyl, phenyl, hydroxy, halogen, nitro, cyano or represents an ether, ester acid or amide radical and X stands for a halide.

13. A compound according to claim 12 wherein $A^1$ and $A^2$ may be the same or different and represent -$COOR^1$, -$CONR^1R^2$, -CN or -$C_6H_5$, $A^3$ represents -H, -OH, -OR, -Cl, -$NO_2$, -CN,-$C_6H_5$, or an alkyl group of one to five carbon atoms, $R^1$ and $R^2$ may be the same or different and represent hydrogen or an alkyl group of one to ten carbon atoms, and R represents an alkyl group of one to ten carbon atoms.

14. An ultraviolet-absorbing composition comprising a compound of the formula

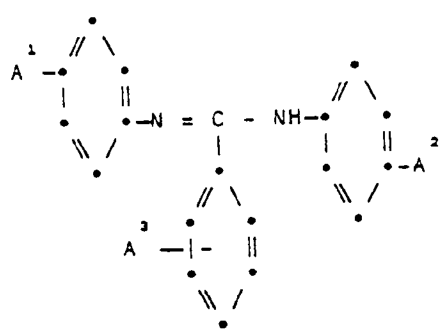

I

wherein
$A^1$ and $A^2$ represent an acid, ester, amide, cyano or aryl radical, $A^3$ represents hydrogen, alkyl, phenyl, hydroxy, halogen, nitro, cyano or represents an ether, ester, acid or amide radical, or a salt thereof, and at least one organic material subject to degradation by ultraviolet light.

15. A composition according to claim 14, wherein $A^1$ and $A^2$ may be the same or different and represent -$COOR^1$, -$CONR^1R^2$, -CN or -$C_6H_5$, $A^3$ represents -H, -OH, -OR, -Cl, -$NO_2$, -CN, -$C_6H_5$, or an alkyl group of one to five carbon atoms, $R^1$ and $R^2$ may be the same or different and represent hydrogen or an alkyl group of one to ten carbon atoms, and R represents an alkyl group of one to ten carbon atoms.

16. A composition according to claim 15 wherein $R^1$ and $R^2$ are methyl or ethyl.

17. A composition according to claim 15 wherein $A^1$ and $A^2$ are both -$COOR^1$.

18. A composition according to claim 17 wherein $A^3$ is hydrogen or -OR.

19. A composition according to claim 18 wherein $R^1$ is methyl or ethyl.

20. A composition according to claim 19 wherein $A^3$ is hydrogen or methoxy.

21. The composition according to claim 20 wherein $R^1$ is ethyl and $A^3$ is hydrogen.

22. The composition according to claim 20 wherein $R^1$ is ethyl and $A^3$ is methoxy.

23. A composition according to claim 20 containing N,N'-bis-(4-ethoxycarbonylphenyl)-2-methoxybenzamidine.

24. A composition according to claim 20 containing N,N'-bis-(4-ethoxycarbonylphenyl)-4-methoxybenzamidine.

25. Process for the manufacture of the compounds of formula I of Claim 1 and their salts, comprising reacting an anilide carrying the p-substituent $A^1$ with a benzotrihalide carrying the substituent $A^3$, or reacting a benzoyl halide carrying the substituent $A^3$ with an anilide carrying a p-substituent $A^2$ and reacting the amide so obtained with a further anilide carrying the p-substituent $A^1$.

26. The use of the benzamidines of formula I of claim 1 or salts thereof as ultraviolet light absorbers.

## Patentansprüche

1. Eine Verbindung der Formel

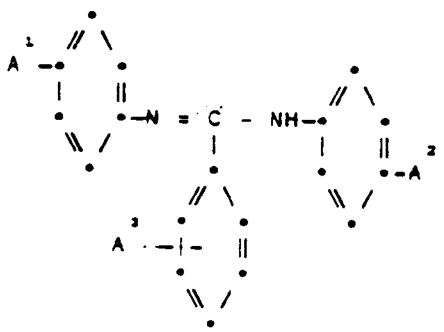

I

worin $A^1$ und $A^2$ gleich oder verschieden sind, und eine Säure, einen Ester, ein Amid, Cyano oder Aryl bedeuten, $A^3$ Wasserstoff, Alkyl, Phenyl, Hydroxy, Halogen, Nitro, Cyano oder einen Aether, einen Ester, eine Säure, ein Amid oder ein Salz davon darstellt.

2. Eine Verbindung gemäss Anspruch 1, worin $A^1$ und $A^2$ gleich oder verschieden sind und $-COOR^1$, $-CONR^1R^2$, $-CN$ oder $-C_6H_5$, $A^3$ $-H$, $-OH$, $-OR$, $-Cl$, $-NO_2$, $-CN$, $-C_6H_5$, oder $C_{1-5}$-Alkyl, $R^1$ und $R^2$ gleich oder verschieden sind, und Wasserstoff oder $C_{1-10}$-Alkyl und R $C_{1-10}$-Alkyl darstellen.

3. Eine Verbindung gemäss Anspruch 2, worin $R^1$ und $R^2$ Methyl oder Aethyl darstellen.

4. Eine Verbindung gemäss Anspruch 2, worin $A^1$ und $A^2$ $-COOR^1$ darstellen.

5. Eine Verbindung gemäss Anspruch 4, worin $A^3$ Wasserstoff oder -OR ist.

6. Eine Verbindung gemäss Anspruch 5, worin $R^1$ Methyl oder Aethyl darstellt.

7. Eine Verbindung gemäss Anspruch 6, worin $A^3$ Wasserstoff oder Methoxy darstellt.

8. Eine Verbindung gemäss Anspruch 7, worin $R^1$ Aethyl und $A^3$ Wasserstoff ist.

9. Eine Verbindung gemäss Anspruch 7, worin $R^1$ Aethyl und $A^3$ Methoxy ist.

10. N,N'-bis-(4-Aethoxycarbonylphenyl)-2-methoxybenzamidin.

**11.** N,N'-bis-(4-Aethoxycarbonylphenyl)-4-methoxybenzamidin.

**12.** Eine Verbindung der Formel

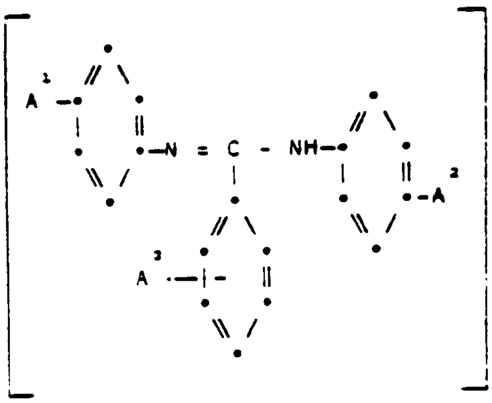

•HX I'

worin $A^1$ und $A^2$ gleich oder verschieden sind, und eine Säure, einen Ester, ein Amid, Cyano oder Aryl bedeuten, $A^3$ Wasserstoff, Alkyl, Phenyl, Hydroxy, Halogen, Nitro, Cyano, oder ein Aether, ein Ester, eine Säure, ein Amid sind, und X für Halogenid steht.

**13.** Eine Verbindung gemäss Anspruch 12, worin $A^1$ und $A^2$ gleich oder verschieden sind und $-COOR^1$, $-CONR^1R^2$, -CN oder $-C_6H_5$, $A^3$ -H, -OH, -OR, -Cl, $-NO_2$, -CN, $-C_6H_5$, oder $C_{1-5}$-Alkyl, $R^1$ und $R^2$ gleich oder verschieden sind und Wasserstoff oder $C_{1-10}$-Alkyl und R $C_{1-10}$-Alkyl darstellen.

**14.** Eine die ultraviolette Strahlung absorbierende Komposition, gekennzeichnet durch einen Gehalt an einer Verbindung der Formel

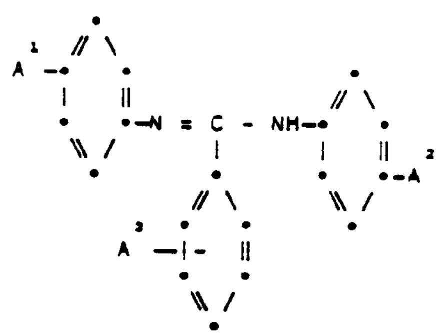

I

worin $A^1$ und $A^2$ gleich oder verschieden sind, und eine Säure, einen Ester, ein Amid, Cyano oder Aryl bedeuten, $A^3$ Wasserstoff, Alkyl, Phenyl, Hydroxy, Halogen, Nitro, Cyano oder ein Aether, ein Ester, eine Säure, ein Amid oder ein Salz davon sind, und X für Halogenid steht, und mindestens einem organischen Material, das durch die Strahlung von ultraviolettem Licht verdorben würde.

**15.** Eine Komposition gemäss Anspruch 14,worin $A^1$ und $A^2$ gleich oder verschieden sind und $-COOR^1$, $-CONR^1R^2$, -CN oder $-C_6H_5$, $A^3$ -H, -OH, -OR, -Cl, $-NO_2$, -CN, $-C_6H_5$, oder $C_{1-5}$-Alkyl, $R^1$ und $R^2$ gleich oder verschieden sind, und Wasserstoff oder $C_{1-10}$-Alkyl und R $C_{1-10}$-Alkyl darstellen.

**16.** Eine Komposition gemäss Anpsruch 15, worin $R^1$ und $R^2$ Methyl oder Aethyl bedeuten.

**17.** Eine Komposition gemäss Anspruch 15, worin $A^1$ und $A^2$ $-COOR^1$ bedeuten.

**18.** Eine Komposition gemäss Anspruch 17, worin $A^3$ Wasserstoff oder -OR ist.

**19.** Eine Komposition gemäss Anpsruch 18, worin $R^1$ Methyl oder Aethyl ist.

20. Eine Komposition gemäss Anspruch 19, worin $A^3$ Wasserstoff oder Methoxy ist.

21. Eine Komposition gemäss Anspruch 20, worin $R^1$ Aethyl und $A^3$ Wasserstoff bedeuten.

22. Eine Komposition gemäss Anspruch 20, worin $R^1$ Aethyl und $A^3$ Methoxy bedeuten.

23. Eine Komposition gemäss Anspruch 20, enthaltend N,N'-bis-(4-Aethoxycarbonylphenyl)-2-methoxybenzamidin.

24. Eine Komposition gemäss Anspruch 20, enthaltend N,N'-bis-(4-Aethoxycarbonylphenyl)-4-methoxybenzamidin.

25. Verfahren zur Herstellung der Verbindungen der Formel I gemäss Anspruch 1 und deren Salze, dadurch gekennzeichnet, dass man ein Anilid mit dem p-Substituenten $A^1$ mit einem Benzotrihalogenid mit den Substituenten $A^3$, oder ein Benzoylhalogenid mit dem Substituenten $A^3$ mit einem Anilid mit p-Substituenten $A^2$ umsetzt, und das so erhaltene Amid mit einem weiteren Anilid, enthaltend den p-Substituenten $A^1$, umsetzt.

26. Verwendung der Benzamidine der Formel I gemäss Anspruch 1 oder deren Salze, als W-Absorber.

## Revendications

1. Un composé de formule

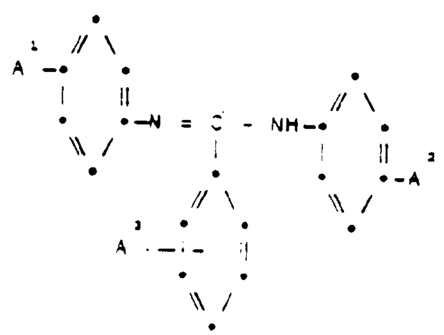

I

dans laquelle
$A^1$ et $A^2$ peuvent être les mêmes ou différents et représentent un radical acide, ester, amide, cyano ou aryle, et $A^3$ représente un hydrogène, alkyle, phényle, hydroxy, halogène, nitro, cyano ou représente un radical éther, ester, acide ou amide, ou un sel de celui-ci.

2. Un composé selon la revendication 1, dans lequel $A^1$ et $A^2$ peuvent être les mêmes ou différents et représentent $-COOR^1$, $-CONR^1R^2$, $-CN$ ou $-C_6H_5$, $A^3$ représente $-H$, $-OH$, $-OR$, $-Cl$, $-NO_2$, $-CN$, $-C_6H_5$, ou un groupement alkyle ayant de un à cinq atomes de carbone, $R^1$ et $R^2$ peuvent être les mêmes ou différents et représentent de l'hydrogène ou un groupement alkyle ayant de un à dix atomes de carbone, et R représente un groupement alkyle ayant de un à dix atomes de carbone.

3. Un composé selon la revendication 2, dans lequel $R^1$ et $R^2$ sont un groupe méthyle ou éthyle.

4. Un composé selon la revendication 2, dans lequel $A^1$ et $A^2$ sont tous deux $-COOR^1$.

5. Un composé selon la revendication 4 dans lequel $A^3$ est un hydrogène ou un -OR.

6. Un composé selon la revendication 5 dans lequel $R^1$ est un groupe méthyle ou éthyle.

7. Un composé selon la revendication 6 dans lequel $A^3$ est un hydrogène ou un groupe méthoxy.

8. Le composé selon la revendication 7 dans lequel $R^1$ est un groupe éthyle et $A^3$ est un hydrogène.

9. Le composé selon la revendication 7 dans lequel $R^1$ est un groupe éthyle et $A^3$ est un groupe méthoxy.

10. N,N′-bis-(4-éthoxycarbonylphényle)-2-méthoxybenzamidine.

11. N,N′-bis-(4-éthoxycarbonylphényle)-4-méthoxybenzamidine.

12. Un composé de formule

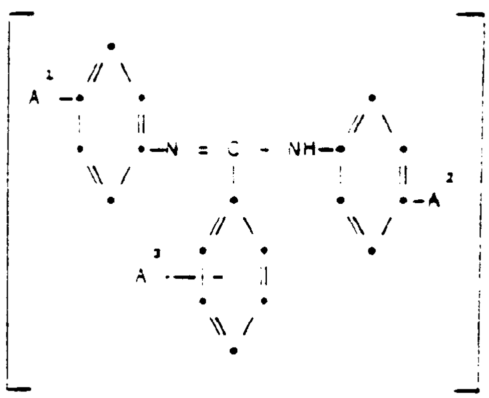

• HX

I'

dans laquelle
$A^1$ et $A^2$ représentent un radical acide, ester, amide, cyano ou aryle, $A^3$ représente un hydrogène, alkyle, phényle, hydroxy, halogène, nitro, cyano ou représente un radical éther, ester, acide ou amide et X désigne un halogénure.

13. Un composé selon la revendication 12, dans lequel $A^1$ et $A^2$ peuvent être les mêmes ou différents et représentent -COOR 1, $-CONR^1R^2$, -CN ou $-C_6H_5$, $A^3$ représente -H, -OH, -OR, -Cl, $-NO_2$, -CN, $-C_6H_5$, ou un groupement alkyle ayant de un à cinq atomes de carbone, $R^1$ et $R^2$ peuvent être les mêmes ou différents et représentent de l'hydrogène ou un groupement alkyle ayant de un à dix atomes de carbone, et R représente un groupement alkyle ayant de un à dix atomes de carbone.

14. Une composition absorbant les ultraviolets comprenant un composé de formule

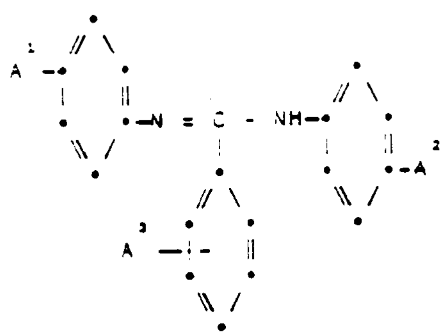

I

dans laquelle
$A^1$ et $A^2$ représentent un radical acide, ester, amide, cyano ou aryle, $A^3$ représente un hydrogène, alkyle, phényle, hydroxy, halogène, nitro, cyano ou représente un radical éther, ester, acide ou amide, ou un sel de celui-ci, et au moins un matériau organique sujet à dégradation par la lumière ultraviolette.

15. Une composition selon la revendication 14, dans laquelle $A^1$ et $A^2$ peuvent être les mêmes ou différents et représentent $-COOR^1$, $-CONR^1R^2$, -CN ou $-C_6H_5$, $A^3$ représente -H, -OH, -OR, -Cl, $-NO_2$, -CN, $-C_6H_5$, ou un groupement alkyle ayant de un à cinq atomes de carbone, $R^1$ et $R^2$ peuvent être les mêmes ou différents et représentent de l'hydrogène ou un groupement alkyle ayant de un à dix atomes de carbone, et R représente un groupement alkyle ayant de un à dix atomes de carbone.

**16.** Une composition selon la revendication 15 dans laquelle $R^1$ et $R^2$ sont un groupe méthyle ou éthyle.

**17.** Une composition selon la revendication 15, dans laquelle $A^1$ et $A^2$ sont tous deux -$COOR^1$.

**18.** Une composition selon la revendication 17 dans laquelle $A^3$ est un hydrogène ou un -OR.

**19.** Une composition selon la revendication 18 dans laquelle $R^1$ est un groupe méthyle ou éthyle.

**20.** Une composition selon la revendication 19 dans laquelle $A^3$ est un hydrogène ou un groupe méthoxy.

**21.** La composition selon la revendication 20 dans laquelle $R^1$ est un groupe éthyle et $A^3$ est un hydrogène.

**22.** La composition selon la revendication 20 dans laquelle $R^1$ est un groupe éthyle et $A^3$ est un groupe méthoxy.

**23.** Une composition selon la revendication 20 contenant de la N,N′-bis-(4-éthoxycarbonylphényle)-2-méthoxybenzamidine.

**24.** Une composition selon la revendication 20 contenant de la N,N′-bis-(4-éthoxycarbonylphényle)-4-méthoxybenzamidine.

**25.** Procédé pour la manufacture des composés de formule I de la revendication 1 et de leurs sels, comprenant la mise en réaction d'un anilide portant $A^1$ comme substituant en position p et d'un benzotrihalogénure portant le substituant $A^3$, ou la mise en réaction d'un halogénure de benzoyle portant $A^3$ comme substituant en position p et d'un anilide portant $A^2$ comme substituant en position p et la mise en réaction de l'amide ainsi obtenue avec un anilide supplémentaire portant $A^1$ comme substituant en position p.

**26.** L'utilisation des benzamidines de formule I de la revendication 1 ou des sels de celles-ci comme absorbeurs de la lumière ultraviolette.